(19) Europäisches Patentamt
European Patent Office
Office européen des brevets

(11) EP 2 305 260 B1

# (12) EUROPEAN PATENT SPECIFICATION

(45) Date of publication and mention of the grant of the patent:
**05.12.2012 Bulletin 2012/49**

(21) Application number: **10181082.8**

(22) Date of filing: **29.07.2005**

(51) Int Cl.:
*A61K 31/472* (2006.01)
*A23L 1/30* (2006.01)
*A61K 31/4725* (2006.01)
*A61P 25/08* (2006.01)
*A61P 25/18* (2006.01)
*A61P 25/20* (2006.01)
*A61P 25/22* (2006.01)
*A61P 25/24* (2006.01)
*C07D 217/24* (2006.01)

## (54) Psychotropic agent and health food containing benzylisoquinoline derivative

Psychotroper Wirkstoff sowie ein Lebensmittel, das ein Benzylisochinolin-Derivat enthält

Agent psychotrope et aliment sain contenant un dérivé de benzylisoquinoline

(84) Designated Contracting States:
**AT BE BG CH CY CZ DE DK EE ES FI FR GB GR HU IE IS IT LI LT LU LV MC NL PL PT RO SE SI SK TR**

(30) Priority: **03.08.2004 JP 2004227227**

(43) Date of publication of application:
**06.04.2011 Bulletin 2011/14**

(62) Document number(s) of the earlier application(s) in accordance with Art. 76 EPC:
**05767135.6 / 1 774 968**

(73) Proprietors:
- **Institute of Oriental Medical Science Inc.**
  **Osaka-shi (JP)**
- **Nakajima, Hiroshi**
  **Uji-shi, Kyoto 611-0041 (JP)**

(72) Inventors:
- **Nakajima, Hiroshi**
  **Uji-shi, Kyoto 6110041 (JP)**
- **Tanahashi, Takao**
  **Nishi-ku, Kobe-shi, Hyogo 6512242 (JP)**
- **Yamada, Jun**
  **Takarazuka-shi, Hyogo 6650881 (JP)**
- **Sun, Shu-Jian**
  **Nishinomiya-shi, Hyogo 6620076 (JP)**
- **Sugimoto, Yumi**
  **Ashiya-shi, Hyogo 6590015 (JP)**

(74) Representative: **Vossius & Partner**
**Siebertstrasse 4**
**81675 München (DE)**

(56) References cited:
**KR-A- 20030 079 104**

- **FUJII T ET AL: "Structure-activity relationships of 4'-O-substituted 1-benzylisoquinolines with respect to their actions on the cell membrane of blood platelets and erythrocytes", EUROPEAN JOURNAL OF PHARMACOLOGY, ELSEVIER BV, NL, vol. 146, no. 2-3, 9 February 1988 (1988-02-09), pages 285-290, XP023750061, ISSN: 0014-2999, DOI: DOI:10.1016/0014-2999(88)90304-4 [retrieved on 1988-02-09]**
- **PROTAIS P ET AL: "Effects of various isoquinoline alkaloids on in vitro 3H-dopamine upta", JOURNAL OF NATURAL PRODUCTS, AMERICAN CHEMICAL SOCIETY, US, vol. 58, no. 10, 1 January 1995 (1995-01-01), pages 1475-1484, XP003005663, ISSN: 0163-3864, DOI: DOI: 10.1021/NP50124A001**
- **DATABASE EMBASE [Online] ELSEVIER SCIENCE PUBLISHERS, AMSTERDAM, NL; 1986, NISHIBE S ET AL: "Alkaloids from embryo of the seed of Nelumbo nucifera", XP009144572, Database accession no. EMB-1986214890 & JOURNAL OF NATURAL PRODUCTS 1986 US, vol. 49, no. 3, 1986, pages 547-548, ISSN: 0163-3864**
- **DATABASE BIOSIS [Online] BIOSCIENCES INFORMATION SERVICE, PHILADELPHIA, PA, US; May 2004 (2004-05), KANG MOONKYU ET AL: "The anti-depressant effect of Nelumbinis semen on the rats under chronic mild stress inducing depression-like symptom", XP002446733, Database accession no. PREV200510186067 & FASEB JOURNAL, vol. 18, no. 8, Suppl. S, May 2004 (2004-05), pages C209-C210, ANNUAL MEETING OF THE AMERICAN-SOCIETY-FOR-BIOCHEMISTRY-AND-MOLE CULAR BIOLOGY/ 8TH CONGRESS OF THE IN; BOSTON, MA, USA; JUNE 12 -16, 2004 ISSN: 0892-6638**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 305 260 B1

- YASUKAWA K ET AL: "Inhibitory effect of cepharanthine and its related compounds on 12-O-tetradecanoylphorbol-13-acetate-induc ed inflammation and inhibition of tumor promotion by cepharanthine in two-stage carcinogenesis in mice, nihon Univ", NIHON UNIVERSITY JOURNAL OF MEDICINE, NIHON UNIVERSITY SCHOOL OF MEDICINE, TOKYO, JP, vol. 31, no. 4, 1 January 1989 (1989-01-01), pages 229-234, XP003005666, ISSN: 0546-0352

## Description

### TECHNICAL FIELD

The present invention relates to a psychotropic agent or a health food for use in preventing and/or alleviating depression, comprising as an effective ingredient, at least one compound selected from the group consisting of armepavine, O-methylarmepavine and pharmaceutically acceptable salts thereof; and use of the effective ingredient as defined above for the manufacture of a psychotropic agent or a health food.

### BACKGROUND ART

Psychiatric disorders are steadily increasing, reflecting the recent stressful society. The psychiatric disorders raise a serious social problem as the patients are inadaptable with society, and also an important issue from the viewpoint of medical economy. Drugs that are capable of affecting mental activity and behavior as the principal pharmacological action are called psychotropic drug, which are classified into schizophrenia drugs, mood-stabilizing drugs, antidepressants, antianxiety drugs, hypnotics, sedative drugs, antiepileptic drugs, alcohol deterrents, antiparkinson drugs, antidementia drugs, and the like. Various drugs with different action mechanisms have been developed and used for treatment of various psychiatric disorders.

For example, anxiety disorders, which are becoming more popular and an important social problem particularly recently, are generally classified into phobic anxiety disorder, agoraphobia, sociophobia, panic disorder, generalized anxiety disorder, obsessive compulsive disorder, certain phobia, and other anxiety disorders. These symptoms often appear in a complicated way.

The symptoms of depression include depressive condition, anhedonia, psychomotor retardation, thinking/cognitive dissonance, anxiety and impatience, autonomic nerve symptom, and the like.

Schizophrenia is a disease wherein integrating ability, an ability to integrate thinking, behavior, and emotion to fit for purpose, deteriorates gradually over an extended period of time and certain hallucination, delusion, and extremely unorganized behavior are observed during the deterioration process, but is rather indistinguishable from depression, withdrawal, maladjustment, or the like, and thus the final diagnosis thereof is made by the symptoms such as hallucination, delusion, and the like. However, the causes of the sickness are yet to be clearly understood, and the claim that schizophrenia is a single disease is still under a cloud.

Examples of the therapeutic drugs for the symptoms above are antidepressants currently used including tricyclic antidepressants such as imipramine, clomipramine, and trimipramine; tetracyclic antidepressants such as maprotiline and mianserin; triazolopyridine-based trazodone; benzketoxime-based selective serotonin reuptake inhibitor (SSRI) fluvoxamine; and the like.

Examples of the sleeping drugs include barbituric hypnotics used for quite a long time; benzodiazepine-based hypnotics such as triazolam, etizolam, brotizolam, flunitrazepam, nitrazepam, quazepam, zopiclone and zolpidem which have been developed more recently; and the like. Further, favorable examples of the antianxiety drugs include the benzodiazepine-based drugs above, fluvoxamine and paroxetine, which are called SSRIs, and the like.

Drugs that have an action on the intracerebral dopamine nervous system have been used for treatment of schizophrenia, but also raised problems of extrapyramidal tract disorders (parkinsonian syndromes) as the adverse reactions, i.e., the adverse reactions observed as catalepsy in animal experiment systems. More recently, atypical antipsychotic drugs, which have an action also on the serotonin nervous system, were introduced and proven to be more effective in treating the disease. Other therapeutic drugs which have been used for treatment are called minor tranquilizers, which act on the cerebral limbic system in the brain such as thalamus, hippocampus, amygdala nidus, and the like and are said to alleviate selectively emotional disorders such as anxiety and stress and stabilize autonomic activities. Typical therapeutic drugs thereof are benzodiazepine compounds and azapyrone-based compounds. However, these drugs also carry the problem of side reactions such as motor coordination disorder, induction of catalepsy, and convulsive action induced by strychnine or picrotoxin.

However, it is pointed out that tricyclic or similar cyclic antidepressants cause anticholinergic actions such as dry mouth, eye adjustment disorder (misty vision), constipation, and dysuria, increase in body weight presumably caused by the antihistamic action, adrenolytic actions such as hypotension, dizziness, and stagger, adverse reactions such as cardiotoxic diseases, as well as acute poisoning by excessive intake.

It is also pointed out that the SSRI-group drugs may cause serotonin syndromes, although the adverse reactions thereof are significantly suppressed.

On the other hand, Saint John's Wort, for example, is famous as a health food for alleviating the various symptoms caused by the abnormality in the central nervous system, and said to be useful in the treatment of depression (Lindley et al., British Medical Journal, 313 (1996) 253). Saint John's Wort, also called rose of Sharon, has long been used as a therapeutic drug for treatment of wound and neuralgia. Recently, it is widely used in the form of health food

tablet mainly in Europe and North America as well as in Japan.

However, a component contained in Saint John's Wort is known to have a serious adverse reaction, photohypersensitivity, and another component a side reaction affecting the kinetics of cyclosporine metabolism in the body.

Next, analgesic agents are known to include narcotic analgesic agents and antipyretic analgesic agents. Narcotic analgesic agents including morphine as a typical example possess a strong analgesic efficacy and are effective for intense pains such as cancer pain and postoperative pain. Narcotic analgesic agents including morphine exhibit a strong analgesic action through opioid receptor in central nervous system but have a problem that they are likely to cause a drug dependence. Antipyretic analgesic agents possess less analgesic efficacy than that of morphine and are null for visceralgia but have no drug dependence. For this reason, antipyretic analgesic agents are widely used for toothache, headache, neuralgia, arthritis, and the like. Antipyretic analgesic agents are known to include agents having no antiinflammatory action which are represented by acetoaminophen having an acting site on central nervous system, and nonsteroidal antiinflammatory agent (NSAID) having both antiinflammatory action and analgesic action such as aspirin. It is considered that the action mechanism of nonsteroidal antiinflammatory agent is to hinder biosynthesis of prostaglandins. However, non-steroidal antiinflammatory agents are prone to frequently cause side effects such as stomach disorders with increasing efficacy.

Lotus (Nelumbo nucifera Gaertner), a plant in water-lily family, has been widely used from root to flower as the ingredients for herbal cuisines from ancient days. In particular, lien tzehsin, green embryo of mature seed (Nelumbinis embryo), has been used in the oriental medicine for treatment of febrile diseases, care-laden vomiting of blood, and oneirogmus as it is said to have functions to reduce fevers, stop bleeding, slow ejaculation, and others ("Encyclopedia of Japanese and Chinese Medicines", Tsuneo Namba, p. 216 and "Comprehensive Dictionary of Chinese Medicines", Ed., Shanghai Science and Technology Publication and Shogakkan, (1985) p. 2750).

In studies on the active components mainly centered on the alkaloids of lien tzehsin, data on structural determination of the benzylisoquinoline and bisbenzylisoquinoline alkaloids were reported (Furukawa et al., Yakugaku Zasshi, 84 (1965) p. 335, Tomita et al., Chemical Pharmaceutical Bulletin, 13 (1965) p. 39 and Kunitomo et al., Phytochemistry, 12 (1973) p. 669), but no pharmacological effects of the alkaloids were reported except a kind of bisbenzylisoquinoline alkaloid, neferine.

On the other hand, with respect to recent studies on pharmacological effects of lien tzehsin, Seibu et al. found the defervescent, antihypertensive, and antipsychotic actions thereof short time ago, but did not show any detailed grounds for the actions (Seibu et al., Journal of Natural Products, 49 (1986) p. 547). Specifically, Seibu et al. only disclosed (1) purification and isolation of four alkaloids from lien tzehsin, (2) animal tests concerning the antihypertensive activity, and (3) that one of the four alkaloids, neferine, exhibited an antihypertensive action, but there were no grounds for the defervescence and antipsychotic actions and no specific data concerning the antihypertensive activity (Seibu et al., Journal of Natural Products, 49 (1986) p. 547).

In addition, the following two studies were reported at the meeting of the Pharmaceutical Society of Japan in 2002. Sato et al. reported from their rat studies that lien tzehsin had a thermoregulating function (Sato et al., Abstract of the 123rd annual meeting of the Pharmaceutical Society of Japan, No. 2, p. 112), while Kawashima et al. an anti-osteoporosis action of a lien tzehsin extract in the human osteoblast system (Kawashima et al., Abstract of the 123rd annual meeting of the Pharmaceutical Society of Japan No. 2, p. 146).

On the other hand, the inventors reported from mice studies that a component of lien tzehsin, neferine, exhibited a locomotor activity-inhibiting action (Ito et al., Abstract of the 50th annual meeting of the Japanese Society of Pharmocognosy, p. 116).

The structure activity relationships of 4'-O-substituted 1-benzylisoquinolines with respect to their actions on the cell membrane of blood platelets and erythrocytes was reported (T. Fujii, et al., European Journal of Pharmacology, 146 (1988), 285-290).

The effects of various isoquinoline alkaloids on in vitro $^{3}$H-dopamine uptake by rat striatal synaptosomes was reported (P. Protais, et al., Journal of Natural Products, 58(10) (1995), 1475-1484).

The isolation of lapachol from *Diphysa Robinoides* was reported (L. Sagrero-Nieves, Journal of Natural Products, 49 (1986), 547).

KR 2003-0079104 A discloses a Nelumbinis semen extract having antidepressive activity, which is prepared by extracting Nelumbinis semen with an alcohol or an alcohol solution.

The anti-depressant effect of Nelumbinis Semen on the rats under chronic mild stress inducting depression-like symptom was investigated (K. Moonkyu et al., Annual Meeting of the American Society for Biochemistry and Molecular Biology, 8th Congress, Boston, MA, USA, 2004).

The inhibitory effect of cepharanthine and its related compounds on 12-O-tetradecanoylphorbol-13-acetate-induced inflammation and inhibition of tumor promotion by cepharanthine in two-stage carcinogenesis in mice was reported (K. Yasukawa, et al., Nihon Univ. J. Med. 31(4) (1989), 229-234).

However, there are no known reports about the pharmacological effects of lien tzehsin components other than those described above.

DISCLOSURE OF INVENTION

An object of the present invention is to provide a psychotropic agent and a health food in which a natural product-derived substance safer even when taken for an extended period of term is used, in particular a psychotropic agent and a health food having an action of preventing and/or alleviating depression.

As a result of extensive investigation for a safer substance acting on the central nervous system and a safer substance having analgesic action or antiinflammatory action for the purposes above, the inventors have found a substance which is higher in safety and has psychotropic action and a substance which is higher in safety and has analgesic and antiinflammatory action among the benzylisoquinoline and bisbenzylisoquinoline derivatives extracted from lien tzehsin, and thus achieved the present invention.

The present invention relates to the following embodiments:

1. A psychotropic agent for use in preventing and/or alleviating depression the agent comprising, as an effective ingredient, at least one compound selected from the group consisting of armepavine, O-methylarmepavine and pharmaceutically acceptable salts thereof.

2. Use of at least one compound selected from the group consisting of armepavine, 0-methylarmepavine and pharmaceutically acceptable salts thereof, for the manufacture of a psychotropic agent for preventing and/or alleviating depression.

3. A health food for use in preventing and/or alleviating depression comprising, as an effective ingredient, at least one compound selected from the group consisting of armepavine, O-methylarmepavine and pharmaceutically acceptable salts thereof.

4. Use of at least one compound selected from the group consisting of armepavine, O-methylarmepavine and pharmaceutically acceptable salts thereof, for the manufacture of a health food for preventing and/or alleviating depression.

5. The health food of item 3 or the use of item 4, wherein the health food is a beverage or drinkable preparation.

Accordingly, the present invention provides the above-mentioned psychotropic agent or health food which agent or food contains a compound as defined above derived from derived from lien tzehsin.

The psychotropic agent comprises armepavine, O-methylarmepavine, or pharmaceutically acceptable salts thereof as effective ingredient, which is selected from the group consisting of a benzylisoquinoline derivative represented by general formula (I):

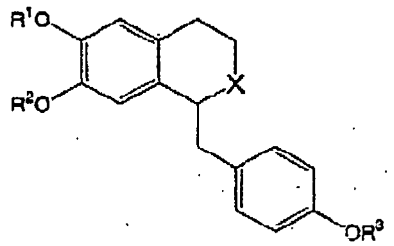

wherein $R^1$, $R^2$ and $R^3$ each independently represent a hydrogen atom, a $C_{1-6}$ alkyl group which may be substituted, an aryl group which may be substituted or a heteroaryl group which may be substituted, and X represents $NR^4$ or $N^+R^5R^6Y$ in which $R^4$ represents a hydrogen atom or a $C_{1-6}$ alkyl group, $R^5$ and $R^6$ each independently represent a hydrogen atom or a $C_{1-6}$ alkyl group, and Y represents a halide, hydroxide, or sulfate ion; and a pharmaceutically acceptable salt thereof.

The benzylisoquinoline derivative represented by the general formula (I) including armepavine, O-methylarmepavine and pharmaceutically acceptable salts thereof has an action of preventing and/or alleviating depression and optionally at least one symptom selected from the group consisting of schizophrenia, anxiety disorder, dysthymia, manic state, epilepsy, and sleep disorder, and/or, an action of effectuating sedation.

The psychotropic agent of the present invention can also optionally comprise, as an effective ingredient, at least one compound selected from the group consisting of a bisbenzylisoquinoline derivative represented by general formula (II):

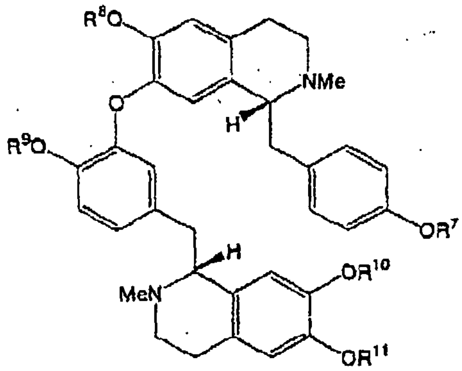

wherein $R^7$, $R^8$, $R^9$, $R^{10}$ and $R^{11}$ each independently represent a hydrogen atom, a $C_{1-6}$ alkyl group which may be substituted, an aryl group which may be substituted or a heteroaryl group which may be substituted; and
a pharmaceutically acceptable salt thereof.

Examples of the compound represented by general formula (II) are neferine:

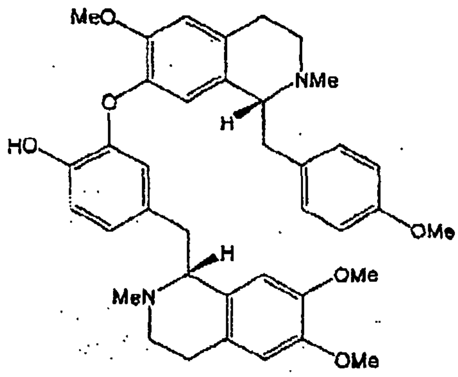

,

liensinine:

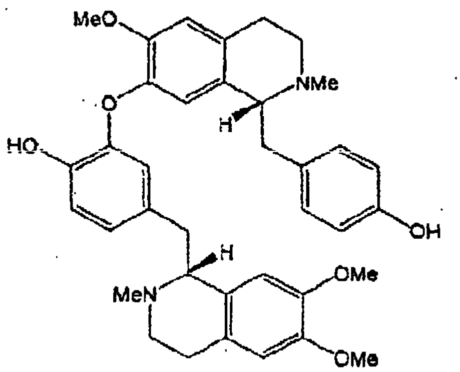

,

and isoliensinine:

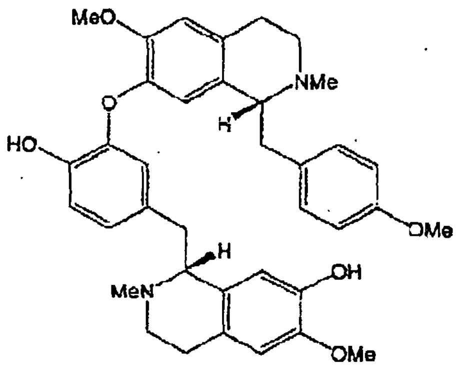

The compound represented by general formula (II) can prevent alleviate depression and optionally a symptom selected from the group consisting of schizophrenia, anxiety disorder, dysthymia, manic state, epilepsy and sleep disorder.

The at least one compound selected from the group consisting of a benzylisoquinoline derivative represented by general formula (I):
the bisbenzylisoquinoline derivative represented by general formula (II): ; and
a pharmaceutically acceptable salt thereof
also possess an

analgesic action and/or antiinflammatory action such as an analgesic action based on an antiinflammatory action.

The compound represented by general formula (I); the compound represented by general formula (II); and a salt thereof can also prevent and/or alleviate a pain and/or an inflammation.

The benzylisoquinoline and bisbenzylisoquinoline derivatives derived from lien tzehsin of which the activity was found in the present invention exhibit no toxicity to the living body and can provide safer drugs having preventive and/or therapeutic effects on central nervous system diseases and preventive and/or therapeutic effects on pains and/or inflammations. They can also be used in health foods and health beverages.

Further, the psychotropic drug according to the present invention is extremely advantageous as it has smaller adverse reactions than conventional drugs and shows activities in a wider range of psychiatric disorders and neurotropic actions.

Moreover, the agent which can possess analgesic action and/or antiinflammatory action is extremely advantageous as it has smaller adverse reactions than conventional drugs and shows activities in a wider range of pains and inflammations.

BEST MODE FOR CARRYING OUT THE INVENTION

In the compounds represented by general formula (I) or (II) above, the $C_{1-6}$ alkyl group in "a $C_{1-6}$ alkyl group which may be substituted" is, for example, a straight-chain or branched-chain alkyl group having to 6 carbons such as a methyl, ethyl, propyl, isopropyl, n-buryl, t-butyl, pentyl, hexyl, or other group. The substituents for these $C_{1-6}$ alkyl group include, for example, halogen atoms. The "$C_{1-6}$ alkyl group which may be substituted" is preferably a methyl group, from the viewpoints of its presence in natural lien tzehsin in a greater amount and the long dietary habits.

In the compounds represented by general formula (I) or (II) above, the aryl group in "an aryl group which may be substituted" is a phenyl, naphthyl, anthryl, or other group. The substituents for these aryl groups include, for example, halogen atoms, $C_{1-6}$ alkyl groups and the like, and examples of the substituted aryl groups include tolyl, phenethyl, and other groups.

Examples of the heteroaryl groups used in "a heteroaryl group which may be substituted" include furyl, thiophenyl, pyrrolyl, thiazolyl, isothiazolyl, oxazolyl, isooxazolyl, imidazolyl, mazolyl, pyridyl, pyrimidyl, pyrazonyl, quinolyl, benzothiophenyl, and morpholinyl groups, and the like. The substituents for these heteroaryl groups include, for example, halogen atoms, $C_{1-6}$ alkyl groups, and the like.

The benzylisoquinoline derivative according to the present invention is a compound selected from

Armepavine

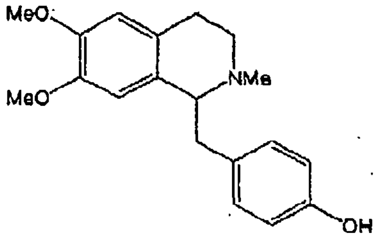

,

and

O-Methylarmepavine

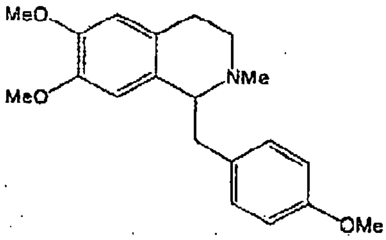

Typical examples of further benzylisoquinoline derivatives include, but are not particularly limited to:

N-Methylcoclaurine

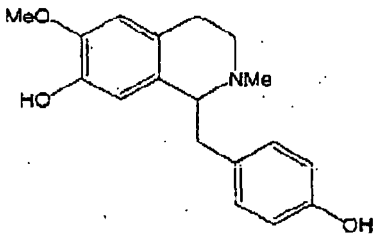

,

N-Methylisococlaurine

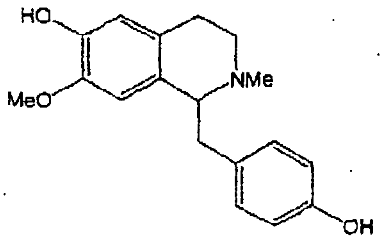

,

N-Norarmepavine

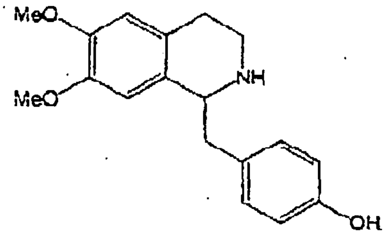

,

O,O-Dimethylcoclaurine,

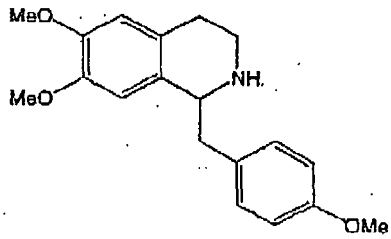

,

Lotusine

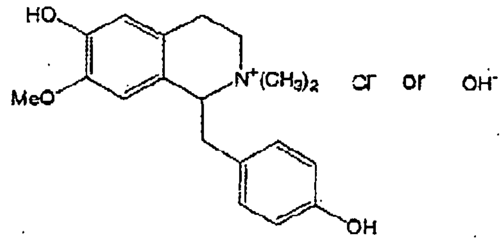

,

, and

Demethylcoclaurine

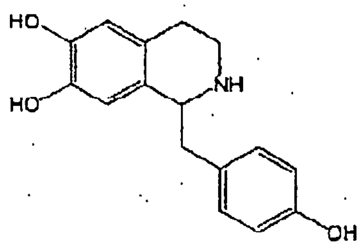

.

Typical examples of the bisbenzylisoquinoline derivatives include, but are not particularly limited to:

Neferine

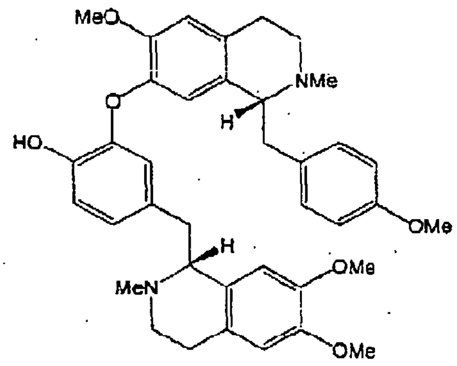

,

Liensinine

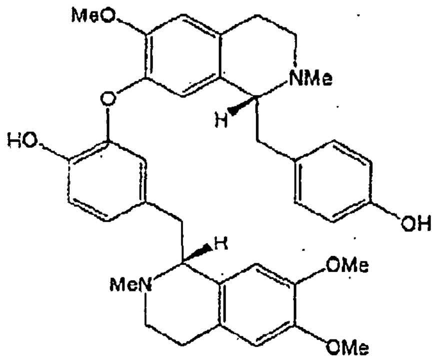

,

and

Isoliensinine

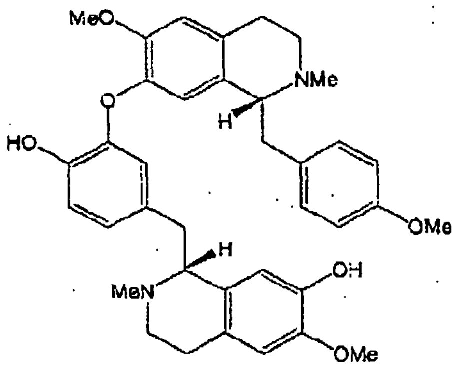

,

Further, the salt of the compound represented by formula (I) or (II) is any one of the pharmaceutically allowable salts thereof. The pharmaceutically allowable salt is any one of the salts with inorganic and organic bases and inorganic and organic acids, and typical examples thereof include salts such as hydrochloride, gluconate, p-toluenesulfonate, fumarate, citrate, succinate, salicylate, oxalate, lactate, hydrobromide, phosphate, methanesulfonate, benzenesulfonate, pamoate, benzoate, tartrate, nitrate, maleate, di-p-toluoyltartrate, acetate, sulfate, hydroiodide, mandelate, and the like.

The compounds represented by formulae (I) and (II) contained in the drugs (psychotropic agent, and analgesic agent and/or antiinflammatory agent) and health foods can be prepared according to the method same as or similar to those described in T. Kametani, S. Takano, J. Chem. Soc. (C), 1969, 298-300 and T. Kametani, H. Yagi, S. Kaneda,

Chem. Pharm. Bull. 14 (9), 947-980, (1966).

For example, the compounds represented by formula (I) can be prepared by reacting phenethylamine and phenylacetic acid respectively having a corresponding substituent into an amide, and cyclizing the amide with phosphorus oxychloride or the like into an isoquinoline skeleton. In addition, a substituent may be introduced on the amine in the isoquinoline skeleton if needed according to any one of the known methods.

The compound represented by formula (II) can be prepared, for example, by condensing two molecules of a derivative having an isoquinoline skeleton, i.e., a compound represented by Formula (I), in a coupling reaction such as copper-catalyzed Ullmann reaction or the like into a compound represented by formula (II) having a bisisoquinoline skeleton.

Alternatively, the compound represented by formula (I) or (II) is an extract of lien tzehsin prepared by a conventional extraction technique from natural products.

In general, the compounds represented by formula (I) may be used alone, or in combination of two or more compounds and the compounds represented by formula (II) may also be used alone or in combination of two or more compounds. Further, the compound represented by formula (I) and the compound represented by formula (II) may be used in combination. According to the present invention at least one compound selected from armepavine, O-methylarmepavine and pharmaceutically acceptable salts thereof must be present.

If the compound is to be isolated by extraction, lien tzehsin is, for example, dried and then extracted with an organic solvent such as methanol, ethanol, aqueous ethanol, hexane or chloroform under heat, and the extract is concentrated. The compound may be purified and isolated by applying the extract thus obtained onto an absorbent such as silica gel and eluting with a chloroform/methanol mixture as the eluent, The extraction fraction containing the compound represented by formula (I) or (II) may be used as it is as a mixture without further purification as the benzylisoquinoline or dibenzylisoquinoline derivative.

In this manner, compounds prepared by synthesis or extracted and isolated from lien tzehsin may be used as the compound represented by formula (I) or (II) .

In addition, the compound represented by formula (I) or (II) may be used as an active ingredient, or alternatively, a lien tzehsin extract containing the compounds may also be used as the active ingredient,

Lien tzehsin containing these benzylisoquinoline and bisbenzylisoquinoline derivatives has been taken for a long time as described above and thus proven to be a safe food. Lien tzehsin is available from many Chinese herbal medicine shops, for example, from Shanghai Traditional Chinese Medical Co., Ltd. (110 Yan An Road, Shanghai, China) or others.

Methods of administering the psychotropic drug according to the present invention are not particularly limited and include oral, intravenous, intramuscular, subcutaneous, percutaneous, transmucosal, intraperitoneal, and other administrations, and oral administration is preferably as it is more convenient for the human being who takes.

A variety of dosage forms are possible as the dosage form of the psychotropic drug according to the present invention, but a dosage form allowing oral administration is preferable. Examples of the dosage forms include, but are not limited to, tablet, capsule, powder, granule, solution, spirit, syrup, and the like. In addition, various pharmaceutically allowable additives may be added to the formulation, and examples thereof include, but are not limited to, excipient, flavor, colorant, sweetener, emulsifier, coating agent, vitamin C, antioxidant, and the like.

The dosage of the psychotropic drug according to the present invention for an adult is 0.1 to 200 mg, preferably 0.1. to 100 mg per day in the case of a benzylisoquinoline derivative, and 1 to 2,000 mg, preferably 1 to 1,000 mg in the case of a bisbenzylisoquinoline derivative. Needless to say, the dosage may be altered according to the age, body weight, symptom, administration method, and the like of the individual who is administered.

The drug may be divided and administered several times a day. In addition, the drug may be administered in combination with another antidepressant or therapy.

The psychotropic drug containing the benzylisoquinoline derivative according to the present invention is effective in preventing and/or alleviating depression and further optionally the symptoms selected from the group consisting of schizophrenia, , anxiety disorder, dysthymia, manic state, epilepsy and sleep disorder, and can have a sedative action.

Methods of administering an analgesic agent and/or an antiinflammatory agent are not particularly limited and include oral, intravenous, intramuscular, subcutaneous, percutaneous, transmucosal, intraperitoneal, and other administrations, and oral administration is preferably as it is more convenient for the human being who intakes.

A variety of dosage forms are possible as the dosage form of the analgesic agent and/or the antiinflammatory agent, but a dosage form allowing oral administration is preferable. Examples of the dosage forms include, but are not limited to, tablet, capsule, powder, granule, solution, spirit, syrup, and the like. In addition, various pharmaceutically acceptable additives may be added to the formulation, and examples thereof include, but are not limited to, excipient, flavor, colorant, sweetener, emulsifier, coating agent, vitamin C, antioxidant, and the like.

The dosage of the analgesic agent and/or the antiinflammatory agent for an adult is 0.1 to 200 mg, preferably 0.1 to 100 mg per day in the case of a benzylisoquinoline derivative, and 1 to 2,000 mg, preferably 1 to 1,000 mg in the case of a bisbenzylisoquinoline derivative. Needless to say, the dosage may be altered according to the age, body

weight, symptom, administration method, and the like of the individual who is administered.

The drug may be divided and administered several times a day. In addition, the drug may be administered in combination with another analgesic or antiinflammatory agent or therapy.

The analgesic agent and/or the antiinflammatory agent containing the benzylisoquinoline or bisbenzylisoquinoline derivative is effective in preventing and/or alleviating a variety of pains such as headache, toothache, neuralgia, arthralgia, myalgia, dysmenorrhea, bruise pain, postoperative pain and traumatogenic pain. Further, the analgesic agent and/or the antiinflammatory agent is effective in preventing and/or alleviating a variety of inflammatory diseases and pathemas such as arthritis, connective tissue inflammation, gout, rheumatoid arthritis, rheumatopyra, osteoarthritis, postoperative inflammation, traumatogenic pain, and upper respiratory tract infections. In particular, the analgesic agent and/or the antiinflammatory agent is expected to effectively exhibit an analgesic action based on an antiinflammatory action. Therefore, the agent can be effective in preventing and/or treating pains resulting from inflammations.

Another embodiment of the present invention is a health food as defined above.

In the present invention, the term "health foods" is a commonly used term meaning the foods sold by claiming that the food is better for health than common foods. Currently in Japan, there is no particular law governing the so-called health foods, which are mainly regulated by the Food Sanitation Law, the Nutrition Improvement Law, and the Pharmaceutical Affairs Law. In April 2001, a new functional health food system was established, and in addition to the conventional foods for specified health use (specified health foods), a new class of "foods with nutrient functional claims" that allows claims of the nutritional functions of part of vitamins and minerals is ordained. The health foods according to the invention include all of these foods. The "health foods" according to the present invention include "special-purpose foods" for use in specific health use, i.e., nutritional supplement foods which are defined generally as "the foods sold for nutrient supplementation or specific health use, in the form different from ordinary foods such as tablet, capsule, and the like".

The health food according to the present invention may be in any form of, for example, tablet, capsule, powder, granule, solution, spirit, syrup, or the like, if it can be taken orally. In addition, the compound represented by formula (I) or (II) may be processed into various foods and drinks by using it as the raw material. Examples of such foods and drinks include noodle, porridge, risotto, jelly, cookie, soup, and beverage, but are not limited thereto.

In addition, various pharmaceutically allowable additives such as excipient, flavor, colorant, sweetener, emulsifier, coating agent, vitamin C, and antioxidant; minerals such as iron, magnesium, and calcium; vitamins; dietary fibers such as alginic acid; as well as various proteins, lipids, saccharides such as lactose any the like may be added to these foods.

Further, health foods having an action to alleviate the symptoms above triggered by mental stress or the like can be prepared by using American ginseng extract, ginkgo biloba extract, soybean extract, licorice extract, pomegranate extract, yam extract or the like in combination with the health food according to the present invention.

Drinkable preparations are prepared by blending the compound represented by formula (I) or (II) and water as the primary ingredients. The ratio of the compound represented by formula (I) or (II) to water in the mixture is not particularly limited but is suitably in the range of 1:5,000 to 1:1,000,000 by weight, preferably 1:10,000 to 1:100,000.

Health food tablets containing the compound represented by formula (I) or (II) can be prepared by adding a diluent and various additives such as creamy powder, malt sugar, cellulose powder, sugar ester, potassium phosphate, starch glycolic acid sodium, calcined calcium, oyster shell powder, cyclodextrin, magnesium citrate, and the like and tabletting the resulting mixture directly by powder compression method.

The dosage of the health food or beverage according to the present invention is not particularly limited, and may be altered arbitrarily according to the administration form, age, body weight, and symptoms.

Actions of the compound represented by formula (I) or (II) on the central nervous system can be evaluated by the screening tests commonly practiced in the art, i.e., animal testing methods used for evaluation of various actions and pharmacological effects in the field of neurotropic action. For example, sedative action can be evaluated by locomotor activity suppression test [Hirose, A., Kato, T., Shimizu, H., Tanaka, H., Nakamura, N., Katsube, J., Jpn. J. Pharmacol. 53, 321-329 (1990)]; therapeutic action on schizophrenia, methamphetamine-induced locomotor activity suppression test [Hirose, A., Kato, T., Shimizu, H., Tanaka, H., Nakamura, N., Katsube, J., Jpn. J. Pharmacol. 53, 321-329 (1990)]; antidepressive action, forced swimming test [Porsolt, R.D., Bertin, A., Jalfre, M., Arch. Int. Pharmacodyn. 299, 327-336 (1977)]; antianxiety action, elevated plus-maze test [Lister, R.G., Psychopharmacology, 112, 13-20 (1993)]; the therapeutic action on obsessive compulsive disorders, glass ball-covering behavior test [Njung'e, K., Hadley, S.L., Pharmacol. Biochem. Behav., 38, 65-67 (1991)]; sleep disorder improvement action, thiopental sleep enhancing test [Sukma, M., Chaichamtipyuth, C., Murakami, Y., Tohda, M., Matsumoto, K., Watanabe, H., J. Ethopharmacology, 83, 87-94 (2002)].

In addition, adverse reactions often associated with conventional drugs such as decline of motor coordination and catalepsy induction can be evaluated by animal testing Methods such as rotarod test [Souri, E., Sharifzadeh, M., Farasam, H., Gharavi, N., J. Pharm. Pharmacol, 51, 853-855 (1999)] and catalepsy test [Hirose, A., Kato, T., Shimizu, H., Tanaka, H., Nakamura., N., Katsube, J., Jpn. J. Pharmacol. 53, 321-329 (1990)].

The analgesic action and antiinflammatory action of the compound represented by formula (I) or (II) can be

evaluated by the screening tests commonly practiced in the art, i.e., animal testing methods used for evaluation of various actions and pharmacological effects in the field of analgesic action and antiinflammatory action. For example, these actions can be evaluated by animal testing methods such as acetic acid-induced writhing test (Koster, R., Anderson, M., de Beer, E. J., Fed. Proc. 18, 412 (1959)), tail-flick test (D'Amour, F.E., Smith, D.L., J. Pharmacol. Exp. Ther. 72, 74-79 (1941)), formalin paw test (Abbott, F.C., Franklin, K.B.J., Westbrook, R.C., Pain 60, 91-102 (1995)), and histamine-induced paw edema test (Y. Oyanagui, Life Sci., 62, PL241-249 (1998).

EXAMPLES

Hereinafter, the present invention will be described in more detail with reference to the following Preparative Examples and Examples, but it should be understood that the present invention is not particularly limited thereto. Various modifications to the benzylisoquinoline and bisbenzylisoquinoline derivatives according to the invention are possible by persons skilled in the art, but these modifications are also embraced by the present invention.

PREPARATIVE EXAMPLE 1

(Extraction of bisbenzylisoquinoline derivatives)

Lien tzehsin (Shanghai Traditional Chinese Medical Co., Ltd., 110 Yan An Road, Shanghai, China) was first washed thoroughly with water and then dried. 500 g of the dried lien tzehsin was extracted three times with 1 L of ethanol while heated under reflux. The extract thus obtained was filtered and dried under reduced pressure, to give approximately 50 g of paste. Quantitative determination of the bisbenzylisoquinoline derivatives by using the Rf values obtained by thin layer chromatography of the paste obtained (silica gel 60F254, chloroform/ methanol/ ammonia water = 90/9/1) revealed that the contents of neferine, liensinine, isoliensinine, and thalifoline were respectively 0.4 %, 0.1%, 0.1%, and 0.1%. The extract was used in the following Examples as Extract 1.

PREPARATIVE EXAMPLE 2

(Extraction of bisbenzylisoquinoline derivatives)

Lien tzehsin was first washed thoroughly with water and then dried. 1,200 g of the dried lien tzehsin was extracted three times with 2 L of hot methanol. The methanol extract thus obtained was evaporated to dryness under reduced pressure, suspended in water, and extracted with n-hexane and chloroform sequentially. The chloroform-soluble fraction was evaporated to dryness under reduced pressure, to give a solid matter in an amount of approximately 8 g. Then, the solid matter was further purified by the methods described below.

Namely, the solid matter was purified by using a column of 580 mm in length and 46 mm in diameter containing 160 g of silica gel as a packing material. It was fractionated by using a gradient mixed chloroform/methanol solution as the eluent. Solid matter in an amount equivalent to 489 mg was obtained as the 20 % methanol fraction. Quantitative determination of the bisbenzylisoquinoline derivatives by using the Rf values obtained by thin layer chromatography of the paste obtained (silica gel 60F254, chloroform/methanol/ammonia water = 90/9/1) revealed that the contents of neferine, liensinine, and isoliensinine were respectively 4.0 %, 7.0 %, and 1.8 %. The solid matter was used as Extract 2 in the following Examples.

PREPARATIVE EXAMPLE 3

(Isolation of neferine)

Lien tzehsin was first washed thoroughly with water and then dried. 1,200 g of the dried lien tzehsin was extracted three times with 2 L of hot methanol. The methanol extract thus obtained was evaporated to dryness under reduced pressure, suspended in water, and extracted with n-hexane and chloroform sequentially. The chloroform-soluble fraction was evaporated to dryness under reduced pressure, to give of solid matter in an amount of approximately 8 g. Then, the solid matter was further purified by the methods described below.

Namely, the solid matter was purified by using a column of 580 mm in length and 46 mm in diameter containing 160 g of silica gel as a packing material. It was fractionated by using a gradient mixed chloroform/methanol solution as the eluent. Solid matter in an amount equivalent to 2.16 mg was obtained as the 5 % methanol fraction.

The crystalline solid thus obtained had a TLC Rf value of 0.39 (silica gel 60F254, chloroform:methanol:ammonia water = 90:9:1). Hydrogen signals corresponding to two N-methyl and three methoxyl groups and 11 aromatic hydrogen signals were observed in $^1$H-NMR, and together with the results of MS, UV, and IR analysis, the compound was identified

as neferine. Analytical values of the neferine are shown below:

$^1$H-NMR ($CDCl_3$): [δ 2.49, 2.52 (6H, each s)], [δ 3.51, 3.73, 3.80, 3.81 (12H, each s)], [δ 5.96 (1H, s), 6.35 (1H, s), 6.51 (1H, s), 6.54 (1H, d, J=2.0 Hz), 6.64 (1H, s), 6.69 (2H, d, J=8.5 Hz), 6.70 (1H, dd, J=8.0, 2.0 Hz), 6.86 (1H, d, J=8.0 Hz), and 6.90 (2H, d, J=8.5 Hz)].
EI-MS m/z: 624 $[M]^+$, 503, and 206.
UV λmax (MeOH) nm (log ε): 230 sh (4.47), and 283 (4.00).
IR νmax (KBr) $cm^{-1}$: 3421, 1612, and 1510.

PREPARATIVE EXAMPLE 4

(Isolation of liensinine and isoliensinine)

In a similar manner to Preparative Example 3, lien tzehsin was first washed thoroughly with water and then dried. 1,200 g of the dried lien tzehsin was extracted three times with 2 L of hot methanol. The methanol extract thus obtained is evaporated to dryness under reduced pressure, suspended in water, and extracted with n-hexane and chloroform sequentially. The chloroform-soluble fraction was evaporated to dryness under reduced pressure, to give of solid matter in an amount of approximately 8 g. The solid matter was purified by using a column of 580 mm in length and 46 mm in diameter containing 160 g of silica gel as a packing material. It was fractionated by using a gradient mixed chloroform/methanol solution as the eluent. The 20 % methanol elution fraction (488.5 mg) was further subjected to a silica gel chromatography and eluted with a chloroform-methanol. The resulting 7.5 % methanol elution fraction (16.4 mg), 10 % methanol elution fraction (40.8 mg), 15 % methanol elution fraction (74.9 mg), 20 % methanol elution fraction (33.0 mg), and 30 % methanol elution fraction (82.8 mg) were subjected respectively to a preparative thin layer chromatography (developing solvent, chloroform:methanol:ammonium hydroxide = 90:9:1), to isolate neferine (19.7 mg), crude liensinine (41.3 mg), and crude isoliensinine (12.5 mg). These crude compounds were further purified with another similar preparative thin layer chromatography respectively, to give liensinine (34.1 mg) and isoliensinine (8.9 mg). Analytical values thereof are shown respectively below:

Liensinine
$^1$H-NMR ($CDCl_3$): [δ 2.55, 2.59 (6H, each s)], [δ 3.43, 3.84, 3.90 (9H, each s)], [δ 5.71 (1H, s), 6.39 (1H, dd, J=8.0, 2.0 Hz), 6.44 (1H, s), 6.59 (1H, s), 6.69 (1H, s), 6.72 (1H, d, J=8.0 Hz), 6.81 (2H, d, J=8.5 Hz), 6.81 (1H, d, J=2.0 Hz), and 7.00 (2H, d, J=8.5 Hz].
SI-MS m/z: 611 [M+H]+, 503, and 206.
UV λmax (MeOH) nm (log ε): 228 sh (4.43), and 282 (3.96).
IR νmax (KBr) $cm^{-1}$: 3415, 1612, and 1514.

Isoliensinine
$^1$H-NMR ($CDCl_3$): [δ 2.39, 2.51 (6H, each s)], [δ 3.74, 3.80, 3.81 (9H, each s), (δ 6.32 (1H, s), 6.37 (1H, s), 6.46 (1H, s), 6.49 (1H, d, J=2.0 Hz), 6.65 (1H, s), 6.71 (2H, d, J=8.5 Hz), 6.73 (1H, dd, J=8.0, 2.0 Hz), 6.83 (1H, d, J=8.0 Hz), and 6.91 (2H, d, J=8.5 Hz)].
CI-MS m/z: 611 $[M+H]^+$.
UV λmax (MeOH) nm (log ε): 230 sh (4.44) and 283 (4.02).
IR νmax (KBr) $cm^{-1}$: 3394, 1611, and 1514.

PREPARATIVE EXAMPLE 5

(Synthesis of N-norarmepavine)

4.02 g (22.2 mmol) of 3,4-Dimethoxyphenethylamine and 3.37 g (22.2 mmol) of p-hydroxyphenylacetic acid were stirred under argon airflow at 170°C for an hour and 15 minutes. Methanol was added to the cooled reaction solution and the resulting crystals were collected by filtration, to give an amide in an amount of 4.29 g. Into a solution of the amide 4.02 g (12.8 mmol) in 80 mL of acetonitrile, 8.0 mL of phosphorus oxychloride was added dropwise at room temperature, and the mixture was then heated under reflux on an oil bath for 15 minutes. After cooled, the mixture was evaporated under reduce pressure, and the resulting residue was dissolved in 80 mL of methanol. After adjustment of the pH of the mixture to pH 7 to 8 by addition of ammonia water, 800 mg of $NaBH_4$ was added gradually and the mixture was stirred at room temperature for 30 minutes. After evaporation of the solvent under reduced pressure, the resulting alkaloid was extracted with chloroform and purified by column chromatography, to give 2.46 g of N-norarmepavine (yield 64 %). Analytical values thereof are shown below:

mp: 199-201°C (MeOH, hydrochloride). EI-MS m/z: 296 and 192 (100 %). CI-MS m/z: 300 $[M+H]^+$. $^1$H-NMR: δ ($CDCl_3$, 200 MHz) 2.76 (2H, brt, J=6.0 Hz, $H_2$-4), 2.86 (1H, dd, J=14.0, 9.0 Hz, H-α), 2.95 (1H, dt, J=12.5, 6.0 Hz, H-3), 3.15 (1H, dd, J=14.0, 4.0 Hz, H-α), 3.24 (1H, dt, J=12.5, 6.0 Hz, H-3), 3.84, 3.86 (6H, each s, OMe x 2), 4.15 (1H, dd, J=9.0, 4.0 Hz, H-1), 6.59, 6.66 (2H, each s, H-5, 8), 6.65 (2H, d, J=8.5 Hz, H-11, 13), and 7.03 (2H, d, J=8.5 Hz, H-10, 14).

PREPARATIVE EXAMPLE 6

(Synthesis of armepavine)

1.20 g (4.01 mmol) of N-norarmepavine obtained in Preparative Example 5 was dissolved in 45 mL of methanol. After addition of 2.22 mL of formalin, the mixture was stirred at room temperature for 30 minutes, added with 890 mg of $NaBH_4$, and stirred additionally at room temperature for 30 minutes. After evaporation of the solvent under reduced pressure, the resulting alkaloid was extracted with chloroform and purified by column chromatography, to give 1.12 g of armepavine (yield 89 %). Analytical values are shown below:

EI-MS: m/z 311 and 206 (100 %). CI-MS m/z: 314 $[M+H]^+$. $^1$H-NMR: δ ($CDCl_3$, 300 MHz) 2.52 (3H, s, NMe), 2.62 (1H, m, H-4), 2.74 (1H, dd, J=13.5, 8.0 Hz, H-α), 2.78-2.95 (2H, m, H-3, 4), 3.14 (1H, dd, J=13.5, 5.0 Hz, H-α), 3.26 (1H, m, H-3), 3.54 (3H, s, OMe), 3.72 (1H, dd, J=8.0, 5.0 Hz, H-1), 3.83 (3H, s, OMe), 5.99, 6.56 (2H, each s, H-5, 8), 6.63 (2H, d, J=8.5 Hz, H-11, 13), and 6.90 (2H, d, J=8.5 Hz, H-10, 14).

PREPARATIVE EXAMPLE 7

(Synthesis of O,O-dimethylcoclaurine)

8.01 g (44.3 mmol) of 3,4-dimethoxyphenethylamine and 7.34 g (44.2 mmol) of p-methoxyphenylacetic acid were stirred under argon airflow at 170°C for an hour and 30 minutes. Methanol was added to the cooled reaction solution and the resulting crystals are collected by filtration, to give 12.5 g of an amide (yield 86 %), 5.00 g (15.2 mmol) of the amide was dissolved in 100 mL of acetonitrile, and 10.0 mL of phosphorus oxychloride was added dropwise to the mixture at room temperature, and the mixture was heated under reflux on an oil bath for additionally 30 minutes. After the solvent was removed by distillation under reduced pressure. The residue was dissolved in 100 mL of methanol. After adjustment of the solution to pH 7 to 8 by addition of ammonia water, 1.52 g of $NaBH_4$ was added gradually and the mixture was stirred at room temperature for 15 minutes. After removal of the solvent by distillation under reduced pressure, the resulting alkaloid was extracted with chloroform and purified by column chromatography, to give 4.43 g of O,O-dimethylcoclaurine (yield 93 %). Analytical values are shown below:

mp: 185-187°C (MeOH, hydrochloride). EI-MS m/z: 313 $[M]^+$ and 192 (100 %). CI-MS: m/z 314 $[M+H]^+$. $^1$H-NMR: δ ($CDCl_3$, 300 MHz) 2.69 (1H, dt, J=16.0, 6.0 Hz, H-4), 2.77 (1H, brdt, J=16.0, 6.0 Hz, H-4), 2.87 (1H, dd, J=14.0, 9.0 Hz, H-α), 2.92 (1H, dt, J=12.0, 6.0 Hz, H-3), 3.15 (1H, dd, J=14.0, 4.5 Hz, H-α), 3.21 (1H, dt, J=12.0, 6.0 Hz, H-3), 3.80, 3.81, 3.86 (9H, each s, OMe x 3), 4.11 (1H, dd, J=9.0, 4.5 Hz, H-1), 6.59, 6.62 (2H, each s, H-5, 8), 6.87 (2H, d, J=8.5 Hz, H-11, 13), and 7.17 (2H, d, J=8.5 Hz, H-10, 14).

PREPARATIVE EXAMPLE 8

(Synthesis of O-methylarmepavine)

1.51 g (4.82 mmol) of O,O-dimethylcoclaurine obtained in Preparative Example 7 was dissolved in 75 mL of methanol and added with 3.0 mL of formalin, and the mixture was stirred at room temperature for 30 minutes. After addition of 1.21 g of $NaBH_4$, the mixture was further stirred at room temperature for 30 minutes. After evaporation of the solvent under reduced pressure, the alkaloid was extracted with chloroform and purified by column chromatography, to give 1.19 g of O-methylarmepavine (yield 75 %). Analytical values are shown below:

EI-MS: m/z 326 and 206 (100 %). CI-MS: m/z 328 [M+H]+. $^1$H-NMR: δ ($CDCl_3$, 300 MHz) 2.53 (3H, s, NMe), 2.59 (1H, m, H-4), 2.75 (1H, dd, J=13.0, 7.5 Hz, H-α), 2.74-2.89 (2H, m, H-3, 4), 3.14 (1H, dd, J=13.0, 5.0 Hz, H-α), 3.18 (1H, m, H-3), 3.55, 3.77, 3.83 (9H, each s, OMe x 3), 3.67 (1H, dd, J=7.5, 5.0 Hz, H-1), 6.01, 6.55 (2H, each s, H-5, 8), 6.80 (2H, d, J=8.5 Hz, H-11, 13), and 7.01 (2H, d, J=8.5 Hz, H-10, 14).

In the following Examples, all extracts and drugs used were dissolved in physiological saline before use for i.p.

administration and the dose was 0.1 ml of physiological saline per 10 g of mouse body weight in either case. All extracts and drugs were suspended in physiological saline containing 1 % sodium carboxymethylcellulose before use for oral administration and the dose was 0.1 ml of 1 % sodium carboxymethylcellulose-containing physiological saline suspension per 10 g of mouse body weight in either case. Male ICR mice (Japan SLC) having a body weight of 25 to 30 g were used in the tests below unless otherwise stated, and were fed in advance at a room temperature of 23 ± 1°C and a relative humidity of 55 ± 5 % in the environment of a cycle of 12-hour-light from 7 a.m. to 7 p.m. and 12-hour-dark from 7 p.m. to 7 a.m. for 3 to 5 days. The mice were allowed to have water and feed freely.

EXAMPLE 1

(Evaluation of sedative action)

Twenty five mice were grouped into five groups each having five mice, to which 5 mg/kg of N-methylcoclaurine hydrochloride (treatment group 1-1: i.p. administration), 50 mg/kg of neferine hydrochloride (treatment group 1-2: oral administration, and treatment group 1-3: i.p. administration), 50 mg/kg of liensinine hydrochloride (treatment group 1-4: oral administration), or physiological saline (negative control group 1-1: i.p. administration) was administered. The ultromotivity of the mice was analyzed by using a locomotor activity monitoring system (NS-ASO1, Neuroscience Inc.). The ultromotivity measurements were performed at an interval of 5 minutes for 60 minutes, and the ultromotivity was judged from the count at each measuring time. The results are summarized in TABLE 1.

TABLE 1

| Test group | Ultromotivity (count) | |
|---|---|---|
| | 30 minutes | 60 minutes |
| Treatment group 1-1 (N-methylcoclaurine, i.p.) | 250 | 250 |
| Treatment group 1-2 (neferine, oral) | 500 | 750 |
| Treatment group 1-3 (neferine, i.p.) | 500 | 760 |
| Treatment group 1-4 (liensinine, oral) | 600 | 800 |
| Negative control group 1-1 (physiological saline, i.p.) | 900 | 1250 |

The results above indicated that the compounds used in treatment groups exerted an obvious sedative action both in oral and i.p. administration. The results also indicated that a benzylisoquinoline derivative, N-methylcoclaurine, had an extremely higher activity than bisbenzylisoquinoline derivatives, neferine and liensinine.

Similar tests were conducted by i.p. administration of 25 mg/kg of N-norarmepavine (treatment group 1-5, n=6), 25 mg/kg of O,O-dimethylcoclaurine (treatment group 1-6, n=5), 10 mg and 25 mg/kg of armepavine (respectively, treatment group 1-7, n=6, and treatment group 1-8, n=6), 10 mg and 25 mg/kg of O-methylarmepavine (respectively, treatment group 1-9, n=6, and treatment group 1-10, n=6), and physiological saline (negative control group 1-2, n=5). The ultromotivity measurements were performed at an interval of five minutes for 60 minutes, and the ultromotivity was subjected to a significant difference test (Tukey's method) by using the counts at respective measuring times. The results are summarized in TABLE 2.

TABLE 2

| Test group | Ultromotivity (count) | |
|---|---|---|
| | 30 minutes | 60 minutes |
| Treatment group 1-5 (N-norarmepavine) | 792.0* ± 71.8 | 1049** ± 85.5 |
| Treatment group 1-6 (O,O-dimethylcoclaurine) | 723.4* ± 68.6 | 1091** ± 188.8 |
| Treatment group 1-7 (armepavine) | 699.3** ± 126.6 | 1107** ± 135.1 |
| Treatment group 1-8 (armepavine) | 260.0** ± 56.15 | 524.0** ± 149.3 |
| Treatment group 1-9 (O-methylarmepavine) | 708.0** ± 85.5 | 1114** ± 158.6 |
| Treatment group 1-10 (O-methylarmepavine) | 214.3** ± 14.6 | 321.3** ± 30.3 |

(continued)

| Test group | Ultromotivity (count) | |
|---|---|---|
| | 30 minutes | 60 minutes |
| Negative control group 1-2 (physiological saline) | 1098 ± 44.7 | 1769 ± 1.15.8 |
| * $p < 0.05$<br>**$p<0.01$ | | |

The ultromotivities of the mice in treatment, groups were significantly more suppressed than that in control groups.
Treatment groups 1-1 to 1-6 are not according to the invention.

EXAMPLE 2 (Reference)

(Evaluation of sleep-enhancing action)

Actions on thiopental sleep by various compounds were evaluated. Thiopental dissolved in physiological saline was administered intraperitoneally into mice at a dose of 60 mg/kg. 50 mg/kg of neferine hydrochloride (treatment group 2-1, n=9), 50 mg/kg of isoliensinine hydrochloride (treatment group 2-2, n=9), 100 mg/kg of lien tzehsin 2 extract hydrochloride (treatment group 2-3, n=9), only physiological saline (negative control group, n=6), or 1 mg/kg of diazeparn (positive control group, n=9) was administered intraperitoneally into the mice 15 minutes before the thiopental administration. The time when the righting reflex of the mice disappeared after thiopental administration was designated as sleep-induction time and the time when the righting reflex was restored was designated as awakening time, and total sleep time was calculated from these values. The results are summarized in TABLE 3.

TABLE 3

| Test group | Total sleep time (min) |
|---|---|
| Treatment group 2-1 (neferine) | 100 |
| Treatment group 2-2 (isoliensinine) | 120 |
| Treatment group 2-3 (extract 2) | 150 |
| Positive control group (diazepam) | 130 |
| Negative control group (physiological saline) | 20 |

As apparent from the results above, the total sleep times of thiopental sleep were obviously elongated in the administration groups,

EXAMPLE 3 (Reference)

(Evaluation of the effects on mental excitement of the patients with schizophrenia)

The effects or neferine on suppression of the enhancement of ultromotivity by methamphetamine were evaluated.
Fifteen mice were grouped into three groups of five mice, to which 50 mg/kg of neferine hydrochloride (treatment group 3-1), 100 mg/kg of the same (treatment group 3-2), and only physiological saline (negative control group) were administered respectively intraperitoneally. Methamphetamine (1 mg/kg) was administered 15 minutes after the administration. After administration of methamphetamine, each of the mice was placed in a transparent polycarbonate cage (22.5 cm × 33.8 cm × 14.0 cm), where the ultromotivity of the mouse was analyzed for 60 minutes by using a locomotor activity analyzer (NS-AS01, Neuroscience Inc.).
The results are summarized as ultromotivity count in TABLE 4.

TABLE 4

| Test group | Ultromotivity (count) |
|---|---|
| Treatment group 3-1 (neferine 50 mg) | 2600 |

(continued)

| Test group | Ultromotivity (count) |
|---|---|
| Treatment group 3-2 (neferine 100 mg) | 1800 |
| Negative control group (physiological saline) | 3300 |

As apparent from the results, neferine has an obvious action suppressing the ultromotivity enhancement by methamphetamine, which was not found in the control group, suggesting that neferine is effective in treating mental excitement of the patients with schizophrenia.

EXAMPLE 4

(Evaluation of antidepressive action)

Forced swimming tests were conducted according to the method of Porsolt et al. A mouse was placed in a cylindrical glass water tank of 10 cm in diameter and 25 cm in height (water temperature: 23°C) filled with water to a depth of 10 cm and forced to swim therein. 14 mice were grouped into two groups each of seven mice, and 100 mg/kg of neferine hydrochloride (treatment group 4-1) or physiological saline (negative control group 4-1) was administered intraperitoneally to the mice in each group. Each mouse was forced to swim 15 and 30 minutes after administration, and the immobile times during the 6-minute periods immediately after forced swimming were determined.

The results are shown in TABLE 5 as the average of the seven mice in respective groups.

TABLE 5

| Test group | Immobile time (sec) | |
|---|---|---|
| | After 15 minutes | After 30 minutes |
| Treatment group 4-1 (neferine) | 165 | 170 |
| Negative control group 4-1 (physiological saline) | 265 | 250 |

The immobile times of the treatment group were obviously shortened, compared to that of the negativity control group. The forced swimming test is widely used for evaluation of the pharmacological effects of antidepressants, and thus the results above suggests that neferine has an antidepressive action.

Similar tests were repeated by i.p. administration of 25 mg/kg of armepavine (treatment group 4-2, n=7), 25 mg/kg of O-methylarmepavine (treatment group 4-3, n=6) and physiological saline (negative control group 4-2, n=5). The significant difference test was conducted According to Dunnett method. The results are summarized in TABLE 6.

TABLE 6

| Test group | Immobile time (sec) after 15 minutes |
|---|---|
| Treatment group 4-2 (armepavine) | 174.5** ± 13.48 |
| Treatment group 4-3 (O-methylarmepavine) | 152.0** ± 14.35 |
| Negative control group 4-2 (physiological saline) | 225.4 ± 9.5 |
| **p<0.01 | |

As shown in TABLE 6, the immobile times in the treatment groups were both shortened significantly from that of the control group.

Treatment group 4-1 is not according to the invention.

EXAMPLE 5

(Evaluation of antianxiety action)

An elevated plus-maze (Neuroscience Inc.) consisting of 2 arms without high walls and 2 arms enclosed by high walls, which were crossing perpendicular to each other, was used for evaluation. A mouse was placed on the

platform at the center in a position facing toward an arm without walls, and the number of times the mouse entered into both arms and the period of the mouse staying in the arms without walls were determined during a period of 5 minutes. The results were evaluated by the ratio (%) of the number of the mouse entering into the arms without walls to the total number of entering into both arms and by the ratio (%) of the period of the mouse staying in the arms without walls to the test period.

The measurements were performed 30 minutes after intraperitoneal administration of 100 mg/kg of neferine hydrochloride (treatment group 5-1, n=5), 100 mg/kg of lien tzehsin extract 2 hydrochloride (treatment group 5-2, n=5) or physiological saline (negative control group, n=5).

The results are summarized in TABLE 7.

TABLE 7

| Test group | Staying period (%) | Invasion times (%) |
|---|---|---|
| Treatment group 5-1 (neferine) | 33 | 70 |
| Treatment group 5-2 (extract 2) | 19 | 58 |
| Negative control group (physiological saline) | 13 | 47 |

From the results above, the number of the mouse entering into the arms without walls and the period of the mouse staying in the arms without walls were found to be obviously increased, compared to those in the negative control group. The results suggest that neferine had an antianxiety action.

Treatment group 5-1 is not according to the invention.

EXAMPLE 6 (Reference)

(Evaluation of the therapeutic action on obsessive compulsive disorders)

In evaluating glass ball-covering behavior, wood chip for breeding (beta chip) (Oriental Yeast Co., Ltd.) was placed in the transparent cage described in Example 3 to a depth of 5 cm, on which 20 glass balls (diameter: 1.5 cm) were placed evenly.

Twenty-one mice were grouped into three groups each of seven mice, to which 25 mg/kg of neferine hydrochloride (treatment group 6-1), 50 mg/kg of the same (treatment group 6-2), and physiological saline (negative control group) were administered intraperitoneally, respectively. A mouse was placed in the cage 15 minutes after administration, and the number of the glass balls not covered with the wood chip was counted by visual observation at an interval of 10 minutes. The number of balls having 2/3 or more of the surface thereof being embedded in the wood chip when the cage was observed from above was counted as the criteria for judgment.

The results are shown in TABLE 8 as the average of seven mice.

TABLE 8

| Test group | Number of glass balls covered with wood chip | | |
|---|---|---|---|
| | After 10 minutes | After 20 minutes | After 30 minutes |
| Treatment group 6-1 (neferine) | 4 | 7 | 10 |
| Treatment group 6-2 (neferine) | 3 | 6 | 9 |
| Negative control group (physiological saline) | 13 | 15 | 17 |

The numbers of balls hidden in treatment groups were all obviously smaller than that of the negative control group. The test results are known to correlate well to the therapeutic action on obsessive compulsive disorders, indicating that neferine is applicable as a therapeutic drug for obsessive compulsive disorders.

Example 7 (Reference)

(Evaluation of motor coordination)

Tests were performed by using mice administered intraperitoneally with 25 mg/kg of neferine hydrochloride (treatment group 7-1, n=6), 25 mg/kg of liensinine hydrochloride (treatment group 7-2, n=6), and physiological saline

(negative control group, n=6) as well as 5 mg/kg of diazepam (positive control group, n=6) and a rotarod equipment (Ugo Basile, Italy) and a locomotor activity monitoring system (NS-ASO1, Neuroscience Inc.). The rotarod test was performed by placing a mouse on a rod having a diameter of 4 cm rotating at a speed of 8 rpm 15 minutes after drug administration and observing whether the mouse can stay on the rod without falling for three minutes. The individuals that had fallen from the rotating rod in the period of 3 minutes were judged that they are with motor coordination disorder. The mice used in the test were selected from those that did not fall in a preliminary test for three minutes conducted before the test on the same day. In addition, the ultromotivity measurements were performed at an interval of five minutes for 60 minutes, and the ultromotivity results were subjected to a significant difference test (Tukey's method) by using the counts at respective measuring times.

The results are summarized in Table 9.

TABLE 9

| Test group | Number of individuals fallen | Significant difference test of ultromotivity |
|---|---|---|
| Treatment group 7-1 (neferine) | 0 | P < 0.01 |
| Treatment group 7-2 (liensinine) group 7-2 (liensinine) | 0 | In declining trend |
| Positive control group (diazepam) | 5 | P < 0.005 |
| Negative control group (physiological saline) | 0 | - |

These results indicate that both drugs in treatment groups exert a sedative action without inducing a muscle-relaxing action or motor coordination disorder.

EXAMPLE 8

(Evaluation of catalepsy)

Catalepsy tests were performed by using mice administered with 200 mg/kg of lien tzehsin extract 1 hydrochloride (treatment group 8-1, n=6) and 100 mg/kg of extract 2 hydrochloride (treatment group 8-2, n=6), 100 mg/kg of neferine (treatment group 8-3, n=6; not according to the invention), 25 mg/kg armepavine (treatment group 8-4, n=6), and 25 mg of O-methylarmepavine (treatment group 8-5, n=5). The mice in the treatment group 8-1 were fed with chow containing 10 % lien tzehsin extract 1 instead of common chow.

The mice except in the treatment group 8-1 were administered intraperitoneally with respective drugs. After 15 minutes from administration, rods of 5.5 cm in height were supplied respectively to the mice in feeding case for holding, and the number of mice which remained there holding the rod for 30 seconds (indicated by */6) was counted and used as catalepsy positivity.

Physiological saline (negativity control group, n=5) and 1 mg/kg of a drug haloperidol (positive control group, n=5) were used as controls respectively.

As a result, the catalepsy positivities were (0/5) in negative control group, (0/6) in treatment groups 8-1 to 8-3, (0/5) in treatment groups 8-4 to 8-5, and (6/6) in the positive control group. Namely, no catalepsy was observed in the mice of treatment groups.

The results indicate that the drugs used in treatment groups do not induce an adverse reaction of extrapyramidal tract disorders.

EXAMPLE 9 (Reference)

(Evaluation of analgesic action of neferine···acetic acid-induced writhing test-1)

Analgesic action of neferine in subcutaneous administration was evaluated by an acetic acid-induced writhing test.

When acetic acid as a chemical stimulating substance is intraperitoneally administered to a mouse, peritonitis is induced, exhibiting peculiar symptom called writhing (symptom of intermittently making action such as spreading of a hind paw, stretching of an abdominal part, and twisting of a lower half of a body). An acetic acid-induced writhing test is a method wherein analgesic action of a compound is determined utilizing this. The number of this writhing is compared between a physiological saline-administered control group and a test drug-administered group, and suppression of the number of writhing by the test drug is investigated thereby assessing its analgesic action.

Seven to ten mice per group were used. Neferine hydrochloride was subcutaneously administered to mice at their back at a dose of 5 mg/kg, 10 mg/kg, 25 mg/kg, 50 mg/kg or 100 mg/kg and, after 15 minutes, a 0.6 % by weight solution of acetic acid in water was intraperitoneally administered at 0.1mL/10 g body weight. The number of writhing was counted every ten minutes for 30 minutes from immediately after acetic acid administration (0 to 10 min, 10 to 20 min, 20 to 30 min). In addition, the total number of writhing for 30 minutes from immediately after acetic acid administration was also obtained. Regarding the resulting measurements, a significant difference test was performed by a Dunnet multiple comparison test method. The results are shown in TABLE 10.

TABLE 10

| Test group | Number of writhing | | | |
|---|---|---|---|---|
| | 0 - 10 min. | 10 - 20 min. | 20 - 30 min. | 0 - 30 min. |
| 5 mg/kg -administered group | 21.8 ± 2.30 | 19.0 ± 2.14 | 10.7 ± 2.04 | 51.0 ± 5.34 |
| 10 mg/kg -administered group | 12.1 ± 1.91** | 11.6 ± 2.66** | 7.9 ± 3.06 | 31.6 ± 6.15** |
| 25 mg/kg -administered group | 8.9 ± 2.22** | 6.9 ± 1.90** | 3.6 ± 1.19** | 19.4 ± 3.48** |
| 50 mg/kg -administered group | 5.7 ± 1.38** | 5.7 ± 1.38** | 5.3 ± 2.15* | 19.7 ± 3.56** |
| 100 mg/kg -administered group | 4.0 ± 1.22** | 6.7 ± 1.31** | 4.2 ± 1.02** | 14.9 ± 2.16** |
| Negative control group (physiological saline) | 21.6 ± 3.22 | 19.0 ± 2.07 | 8.6 ± 1.33 | 49.2 ± 5.26 |

* $p < 0.05$
** $p < 0.01$

As apparent from TABLE 10, neferine shows significant analgestic action against the negative control group at a dose of 10 mg/kg or more in 15 minute-before subcutaneous administration.

(Evaluation of analgesic action of neferine···acetic acid-induced writhing test-2)

According to the same manner as in acetic acid-induced writhing test-1 except that acetic acid was intraperitoneally administered 30 minutes after subcutaneous administration of neferine in the aforementioned acetic acid-induced writhing test-1, an acetic acid-induced writhing test was performed. However, the dose of neferine was only 50 mg/kg. The results are shown in TABLE 11.

TABLE 11

| Test group | Number of writhing | | | |
|---|---|---|---|---|
| | 0 - 10 min. | 10 - 20 min. | 20 - 30 min. | 0 - 30 min. |
| 50 mg/kg -administered group | 10.6 ± 2.53** | 10.3 ± 1.90** | 6.6 ± 1.53** | 27.4 ± 3.73*** |
| Negative control group (physiological saline) | 24.3 ± 1.90 | 20.9 ± 2.15 | 14.6 ± 2.34 | 59.7 ± 4.70 |

** $p < 0.01$
*** $p < 0.001$

As apparent from TABLE 11, neferine also shows significant analgesic action against the negative control group also in 30 minute-before subcutaneous administration.

(Evaluation of analgesic action of neferine···acetic acid-induced writhing test-3)

Analgesic action of neferine in oral administration was evaluated by an acetic acid-induced writhing test.

According to the same manner as in the acetic acid-induced writhing test-1 except that acetic acid was intraperitoneally administered 30 minutes after oral administration of neferine in the aforementioned acetic acid-induced writhing test-1, an acetic acid-induced writhing test was performed. However, five to six mice per group were used. The dose of neferine was 10 mg/kg and 50 mg/kg. Further, aspirin was used as a positive control drug. The dose of aspirin was 50 mg/kg. The results are shown in TABLE 12.

TABLE 12

| Test group | Number of writhing | | | |
|---|---|---|---|---|
| | 0 - 10 min. | 10 - 20 min. | 20 - 30 min. | 0 - 30 min. |
| 10 mg/kg -administered group | 7.6 ± 1.63** | 9.2 ± 1.35** | 6.5 ± 2.50* | 23.3 ± 1.52** |
| 50 mg/kg -administered group | 2.4 ± 1.94** | 4.0 ± 1.30** | 1.6 ± 0.81** | 8.0 ± 3.08** |
| Positive control group (aspirin, 50 mg/kg) | 10.8 ± 3.41** | 15.2 ± 1.83* | 7.4 ± 1.23* | 33.4 ± 5.35** |
| Negative control group (physiological saline) | 29.6 ± 3.64 | 24.4 ± 2.98 | 12.8 ± 1.74 | 66.8 ± 7.45 |

* $p < 0.05$
** $p < 0.01$

As apparent from TABLE 12, neferine shows strong analgesic action as compared with aspirin in oral administration.

EXAMPLE 10

(Evaluation of analgesic action of armepavine···acetic acid-induced writhing test)

Analgesic action of armepavine in subcutaneous administration was evaluated in an acetic acid-induced writhing test.

According to the same manner as in acetic acid-induced writhing test-1 except that armepavine was used in place of neferine in the aforementioned acetic acid-induced writhing test-1, an acetic acid-induced writhing test was performed. However, the dose of armepavine was 5 mg/kg and 10 mg/kg. The results are shown in TABLE 13.

TABLE 13

| Test group | Number of writhing | | | |
|---|---|---|---|---|
| | 0 - 10 min. | 10 - 20 min. | 20 - 30 min. | 0 - 30 min. |
| 5 mg/kg -administered group | 19.0 ± 3.21 | 14.7 ± 1.92 | 9.4 ± 1.81 | 41.7 ± 5.24* |
| 10 mg/kg -administered group | 8.6 ± 1.41** | 10.7 ± 2.24** | 5.3 ± 1.39 | 24.6 ± 4.23** |
| Negative control group (physiological saline) | 26.4 ± 3.36 | 21.8 ± 2.13 | 8.9 ± 1.09 | 57.1 ± 3.60 |

* $p < 0.05$
** $p < 0.01$

As apparent from TABLE 13, armepavine shows significant analgesic action against the negative control group at a dose of 5 to 10 mg/kg.

EXAMPLE 11

(Evaluation of analgesic action of O-methylarmepavine···acetic acid-induced writhing test)

Analgesic action of O-methylarmepavine in subcutaneous administration was evaluated by an acetic acid-induced writhing test.

According to the same manner as in acetic acid-induced writhing test-1 except that O-methylarmepavine was used in place of neferine in the aforementioned acetic acid-induced writhing test-1, an acetic acid-induced writhing test was performed. However, the dose of O-methylarmepavine was 5 mg/kg and 10 mg/kg. The results are shown in TABLE 14.

TABLE 14

| Test group | Number of writhing | | | |
|---|---|---|---|---|
| | 0 - 10 min. | 10 - 20 min. | 20 - 30 min. | 0 - 30 min. |
| 5 mg/kg -administered group | 11.6 ± 1.41** | 11.2 ± 225** | 7.4 ± 1.52 | 30.2 ± 1.90* |
| 10 mg/kg -administered group | 5.9 ± 1.73** | 4.3 ± 1.19** | 3.1 ± 0.58** | 13.3 ± 1.69** |

(continued)

| Test group | Number of writhing | | | |
|---|---|---|---|---|
| | 0 - 10 min. | 10 - 20 min. | 20 - 30 min. | 0 - 30 min. |
| Negative control group (physiological saline) | 26.4 ± 3.36 | 21.8 ± 2.13 | 8.9 ± 1.09 | 57.1 ± 3.60 |
| ** p< 0.01 | | | | |

As apparent from TABLE 14, O-methylarmepavine shows significant analgesic action against the negative control group at a dose of 5 to 10 mg/kg.

EXAMPLE 12 (Reference)

(Evaluation of analgesic action of neferine···tail flick test)

A tail flick test is a method in which, when radiant heat stimulation is locally added to a part of a tail 1 to 2 cm away from the tail root part of a mouse, a reaction of raising a tail as a provisional pain reaction is manifested, and anti-invasion action is determined by utilizing this reaction.
Seven to nine mice per group were used. Mice were fixed, and radiant heat stimulation was locally added to a part of the tail 1 to 2 cm away from the tail root part using a tail flick unit apparatus (Ugo

EXAMPLE 13 (Reference)

(Evaluation of analgesic action of neferine···formalin pad test)

A formalin pad test is a method in which, when formalin is subcutaneously administered to a pad of a mouse, the mouse cases licking, and analgesic action of a test drug is determined by utilizing this. Suppression of licking in a test drug-administered group was investigated relative to a physiological saline-administered control group, and analgestic action was evaluated.
Six mice per group were used. Neferine hydrochloride was intraperitoneally administered at a dose of 25 mg/kg, 50 mg/kg or 100 mg/kg and, after fifteen minutes, 20 μl of a 0.2 % by weight formalin aqueous solution was subcutaneously administered to a right hind paw pad. After formalin administration, licking was regarded as a provisional pain reaction, and a licking sustaining time in a first phase from immediately after formalin administration to five minutes after formalin administration and in a second phase from 15 minutes to 30 minutes after formalin administration was measured. Regarding the resulting measurements, a significant difference test was performed by a Dunnet multiple comparison test method. The results are shown in TABLE 16.
Basil). The reaction that a mouse raised a tail upon addition of radiant heat was regarded as a provisional pain reaction, and a time until its manifestation (latency time) was measured. Neferine hydrochloride was intraperitoneally administered at a dose of 50 mg/kg or 100 mg/kg. Latency after administration of the test drug was test latency, and the measurement was performed 15 minutes after administration of the test drug. In order to avoid damage of the tail of mice, a cut off time was 10 seconds.

$$\%\ \text{MPE (percent maximum possible effect)} = [(\text{latency of test drug - administered group} - \text{latency of control group})/(10 - \text{average latency of control group})] \times 100$$

Regarding the resulting measurements, a significant difference test was performed by a Dunnet multiple comparison test method. The results are shown in TABLE 15.

TABLE 15

| Test group | % MPE |
|---|---|
| 50 mg/kg -administered group | 5.0 ± 2.30 |
| 100 mg/kg -administered group | 15.6 ± 2.89** |

(continued)

| Test group | % MPE |
|---|---|
| Negative control group (physiological saline) | 0,6 ± 3.02 |
| **p<0.01 | |

As apparent from TABLE 15, neferine significantly extended latency until manifestation of the provisional pain reaction against the negative control group at a dose of 100 mg/kg.

TABLE 16

| Test group | Licking sustaining time (second) | |
|---|---|---|
| | First phase | Second phase |
| 25 mg/kg -administered group | 95.2 ± 3.79 | 235.7 ± 13.01 |
| 50 mg/kg -administered group | 98.7 ± 8.57 | 200.3 ± 24.43* |
| 100 mg/kg -administered group | 78.3 ± 7.57** | 139.7 ± 18.61** |
| Negative control group (physiological saline) | 91.7 ± 9.24 | 268.7 ± 13.67 |
| * p < 0.05<br>** p < 0.01 | | |

As apparent from TABLE 16, in the first phase reaction, suppression of mouse licking was seen only in the neferine 100 mg/kg-administered group. In the Second phase reaction, a significant difference was not seen in the neferine 25 mg/kg-administered group, but in 50 mg/kg and 100 mg/kg-administered groups, suppression of mouse licking was caused in a dose dependent manner.

The first phase in the formalin-induced pain test is a pain reaction due to direct action of formalin itself on a nerval end, and a second phase is a subsequent pain reaction due to inflammation. From results of the aforementioned test regarding neferine, it was recognized that neferine strongly suppressed licking in a second phase based on inflammation. Therefore, from results of the acetic acid-induced writhing test and the tale-flick test (neferine is recognized to have suppression action at 10 mg/kg or more in the acetic acid-induced writhing reaction, but the neferine 100 mg/kg-administered group only shows weak suppression action in the tail-flick test), analgesic action of neferine is probably manifested mainly by induction of anti-inflammatory action.

Inflammation is a tissue or systemic reaction which is caused against invasion into a living body. $PGE_2$ and $PGI_2$ are involved in pain due to inflammation. PGs (prostaglandins) increase a bloodstream amount at an inflammation site, and promote a pain. The aforementioned NSAID suppresses production of PGs, and shows analgesic action. Probably neferine also acts on inflammation reaction mechanism, and induces analgesic action. In addition, when inflammation occurs, histamine, serotonine, and the like are released, and are associated with regulation of algesthesia. It is presumed that analgesic action is manifested also by action of neferine on these substances in a living body.

EXAMPLE 14

(Evaluation of analgesic action of O-methylarmepavine···formalin pad test)

According to the same manner as in Example 13 except that 10 mg/kg of O-methylarmepavine was intraperitoneally administered in place of neferine, a formalin pad test was performed. The results are shown in TABLE 17.

TABLE 17

| Test group | Licking sustaining time (second) | |
|---|---|---|
| | First phase | Second phase |
| 10 mg/kg -administered group | 70.9 ± 11.24 | 126.9 ± 18.4** |
| Negative control group (physiological saline) | 83.2 ± 6.4 | 249.1 ± 23.3 |
| ** p < 0.01 | | |

As apparent from TABLE 17, in O-methylarmepavine 10 mg/kg-administered group, suppression of mouse

licking was not seen at a first phase reaction, but suppression of mouse licking was caused in a second phase reaction.

EXAMPLE 15 (Reference)

(Evaluation of anti-inflammatory action of neferine ···histamine-induced paw edema test)

Six mice per group were used. 100 mg/kg of neferine hydrochloride was intraperitoncally administered, and 10 μg of histamine as a prophlogistic agent was subcutaneously administered to a right hind paw pad after 30 minutes. The thickness of the right hind paw was measured immediately before histamine administration and 12 minutes after histamine administration. The thickness immediately before administration was subtracted from the thickness of the paw after histamine administration, and this was adopted as a thickness of paw edema. The results are shown in TABLE 18. As apparent from TABLE 18, paw edema due to histamine was suppressed by neferine.

TABLE 18

| Test group | Thickness of paw edema (mm) |
|---|---|
| neferine 100 mg/kg -administered group | 0.32 ± 0.03** |
| Negative control group (physiological saline) | 0.68 ± 0.05 |
| ** p < 0.01 | |

EXAMPLE 16

(Evaluation of anti-inflammatory action of O-methylarmepavine ···histamine-induced paw edema test)

According to the same manner as in Example 15 except that 10 mg/kg of O-methylarmepavine was intraperitoneally administered and, after 15 minutes, 10 μg of histamine as a prophlogistic agent was subcutaneously administered to a right hind paw pad, a histamine-induced paw edema test was performed. The results are shown in TABLE 19. As apparent from TABLE 19, paw edema due to histamine was suppressed by O-methylarmepavine.

TABLE 19

| Test group | Thickness of paw edema (mm) |
|---|---|
| O-methylarmepavine 10 mg/kg-administered group | 0.37 ± 0.06** |
| Negative control group (physiological saline) | 0,71 ± 0.04 |
| ** p < 0.01 | |

EXAMPLE 17

(Preparative examples)

(A) Preparation of tablet A mixture of 4 g of neferine, 400 g of oligosaccharide, 30 g of calcium phosphate, and 170 g of sucrose fatty acid ester was blended in a V-type mixer for 20 minutes and then compressed in a rotary press under a pressure of 800 kgf/cm$^2$, to give 100-mg tablets.

(B) Preparative of drinkable preparation

0.1 g of neferine and 20 g of boiled extract of jasmine tea were dissolved in 970 cc of water, and the solution was then filtered.

(C) Preparation of capsule

2 g of neferine previously crushed to an average grain size of about 100 micron with a ball mill and 50 g of lactose were mixed well and the mixture was encapsulated with gelatin to give capsules.

(D) Preparative of powdered formulation:

2 g of neferine and 20 g of American ginseng extract, 10 g of castor oil, and 5 g of Hivis Wako (Wako Pure Chemical Industries) were blended. The mixture was dropped onto a rotating disk, to give powders having a diameter of approximately 30 micron.

## Claims

1. A psychotropic agent for use in preventing and/or alleviating depression the agent comprising, as an effective ingredient, at least one compound selected from the group consisting of armepavine, O-methylarmepavine and pharmaceutically acceptable salts thereof.

2. Use of at least one compound selected from the group consisting of armepavine, O-methylarmepavine and pharmaceutically acceptable salts thereof, for the manufacture of a psychotropic agent for preventing and/or alleviating depression.

3. A health food for use in preventing and/or alleviating depression comprising, as an effective ingedient, at least one compound selected from the group consisting of armepavine, O-methylarmepavine and pharmaceutically acceptable salts thereof.

4. Use of at least one compound selected from the group consisting of armepavine, O-methylarmepavine and pharmaceutically acceptable salts thereof, for the manufacture of a health food for preventing and/or alleviating depression.

5. The health food of claim 3 or the use of claim 4, wherein the health food is a beverage or drinkable preparation.

## Patentansprüche

1. Ein psychotropisches Mittel zur Verwendung bei der Vorbeugung und/oder Linderung von Depression, wobei das Mittel als einen Wirkstoff mindestens eine Verbindung, ausgewählt aus der Gruppe bestehend aus Armepavin, O-Methylarmepavin und pharmazeutisch verträglichen Salzen davon, umfasst.

2. Verwendung mindestens einer Verbindung, ausgewählt aus der Gruppe bestehend aus Armepavin, O-Methylarmepavin und pharmazeutisch verträglichen Salzen davon, zur Herstellung eines psychotropischen Mittels zur Vorbeugung und/oder Linderung von Depression.

3. Ein gesundheitsförderndes Nahrungsmittel zur Verwendung bei der Vorbeugung und/oder Linderung von Depression, umfassend als einen Wirkstoff mindestens eine Verbindung, ausgewählt aus der Gruppe bestehend aus Armepavin, O-Methylarmepavin und pharmazeutisch verträglichen Salzen davon.

4. Verwendung mindestens einer Verbindung, ausgewählt aus der Gruppe bestehend aus Armepavin, O-Methylarmepavin und pharmazeutisch verträglichen Salzen davon, zur Herstellung eines gesundheitsfördernden Nahrungsmittels zur Vorbeugung und/oder Linderung von Depression.

5. Das gesundheitsfördernde Nahrungsmittel gemäß Anspruch 3 oder die Verwendung gemäß Anspruch 4, wobei das gesundheitsfördernde Nahrungsmittel ein Getränk oder ein trinkbares Präparat ist.

## Revendications

1. Un agent psychotrope pour une utilisation dans la prévention et/ou l'atténuement de la dépression, l'agent comprenant, en tant qu'ingrédient efficace, au moins un composé choisi parmi le groupe constitué de l'armepavine, de l'O-methylarmepavine et des sels acceptables sur le plan pharmaceutique de celles-ci.

2. Utilisation d'au moins un composé choisi parmi le groupe constitué de l'armepavine, de l'O-methylarmepavine et des sels acceptables sur le plan pharmaceutique de celles-ci pour la préparation d'un agent psychotrope pour la

prévention et/ou l'atténuement de la dépression.

**3.** Un aliment santé pour une utilisation dans la prévention et/ou l'atténuement de la dépression, l'aliment santé comprenant, en tant qu'ingrédient efficace, au moins un composé choisi parmi le groupe constitué de l'armepavine, de l'O-methylarmepavine et des sels acceptables sur le plan pharmaceutique de celles-ci.

**4.** Utilisation d'au moins un composé choisi parmi le groupe constitué de l'armepavine, de l'O-methylarmepavine et des sels acceptables sur le plan pharmaceutique de celles-ci pour la préparation d'un aliment santé pour la prévention et/ou l'atténuement de la dépression.

**5.** L'aliment santé selon la revendication 3 ou l'utilisation selon la revendication 4, dans lequel ou laquelle l'aliment santé est une boisson ou une préparation buvable.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 20030079104

### Non-patent literature cited in the description


- **LINDLEY et al.** *British Medical Journal,* 1996, vol. 313, 253
- **TSUNEO NAMBA.** Encyclopedia of Japanese and Chinese Medicines. 216
- Comprehensive Dictionary of Chinese Medicines. 1985, 2750
- **FURUKAWA et al.** *Yakugaku Zasshi,* 1965, vol. 84, 335
- **TOMITA et al.** Chemical Pharmaceutical Bulletin. 1965, vol. 13, 39
- **KUNITOMO et al.** *Phytochemistry,* 1973, vol. 12, 669
- **SEIBU et al.** *Journal of Natural Products,* 1986, vol. 49, 547
- *Pharmaceutical Society of Japan,* 2002
- **SATO et al.** *Abstract of the 123rd annual meeting of the Pharmaceutical Society of Japan, No. 2,* 112
- **KAWASHIMA et al.** *Abstract of the 123rd annual meeting of the Pharmaceutical Society of Japan No. 2,* 146
- **ITO et al.** *Abstract of the 50th annual meeting of the Japanese Society of Pharmocognosy,* 116
- **T. FUJII et al.** *European Journal of Pharmacology,* 1988, vol. 146, 285-290
- **P. PROTAIS et al.** *Journal of Natural Products,* 1995, vol. 58 (10), 1475-1484
- **L. SAGRERO-NIEVES.** *Journal of Natural Products,* 1986, vol. 49, 547
- **K. MOONKYU et al.** *Annual Meeting of the American Society for Biochemistry and Molecular Biology, 8th Congress, Boston, MA, USA,* 2004
- **K. YASUKAWA et al.** *Nihon Univ. J. Med.,* 1999, vol. 31 (4), 229-234
- **T. KAMETANI ; S. TAKANO.** *J. Chem. Soc. (C,* 1969, 298-300
- **T. KAMETANI ; H. YAGI ; S. KANEDA.** *Chem. Pharm. Bull.,* 1966, vol. 14 (9), 947-980
- **HIROSE, A. ; KATO, T. ; SHIMIZU, H. ; TANAKA, H. ; NAKAMURA, N. ; KATSUBE, J.** *Jpn. J. Pharmacol.,* 1990, vol. 53, 321-329
- **PORSOLT, R.D. ; BERTIN, A. ; JALFRE, M.** *Arch. Int. Pharmacodyn.,* 1977, vol. 299, 327-336
- **LISTER, R.G.** *Psychopharmacology,* 1993, vol. 112, 13-20
- **NJUNG'E, K. ; HADLEY, S.L.** *Pharmacol. Biochem. Behav.,* 1991, vol. 38, 65-67
- **SUKMA, M. ; CHAICHAMTIPYUTH, C. ; MURAKAMI, Y. ; TOHDA, M. ; MATSUMOTO, K. ; WATANABE, H.** *J. Ethopharmacology,* 2002, vol. 83, 87-94
- **SOURI, E. ; SHARIFZADEH, M. ; FARASAM, H. ; GHARAVI, N.** *J. Pharm. Pharmacol,* 1999, vol. 51, 853-855
- **HIROSE, A. ; KATO, T. ; SHIMIZU, H. ; TANAKA, H. ; NAKAMURA., N. ; KATSUBE, J.** *Jpn. J. Pharmacol.,* 1990, vol. 53, 321-329
- **KOSTER, R. ; ANDERSON, M. ; DE BEER, E. J.** *Fed. Proc.,* 1959, vol. 18, 412
- **D'AMOUR, F.E. ; SMITH, D.L.** *J. Pharmacol. Exp. Ther.,* 1951, vol. 72, 74-79
- **ABBOTT, F.C. ; FRANKLIN, K.B.J. ; WESTBROOK, R.C.** *Pain,* 1995, vol. 60, 91-102
- **Y. OYANAGUI.** *Life Sci.,* 1998, vol. 62, PL241-249